# EUROPÄISCHE PATENTSCHRIFT

(11) **EP 3 944 827 B1**
(45) Veröffentlichungstag und Bekanntmachung des Hinweises auf die Patenterteilung: **11.01.2023**
(21) Anmeldenummer: 20188396.4
(22) Anmeldetag: 29.07.2020
(51) Int. Cl.: A61B 17/29, A61B 17/30

(54) **CHIRURGISCHES INSTRUMENT MIT EINER PUSH-PUSH-VERRIEGELUNGSMECHANIK**
SURGICAL INSTRUMENT HAVING A PUSH-PUSH LOCKING MECHANISM
INSTRUMENT CHIRURGICAL À MÉCANIQUE DE VERROUILLAGE PUSH-PUSH

(43) Veröffentlichungstag der Anmeldung: 02.02.2022
(73) Patentinhaber: Tschida - Kelch, Ivonne, 78580 Bärenthal (DE)
(72) Erfinder: Tschida, Peter, 78580 Bärenthal (DE)
(74) Vertreter: LBP Lemcke, Brommer & Partner Patentanwälte mbB

(56) Entgegenhaltungen:
- EP-A1- 2 792 319
- DE-A1- 10 314 072
- DE-C1- 19 841 249
- US-A- 5 618 306
- US-A1- 2013 247 333
- US-A1- 2018 153 535
- US-B1- 6 322 578

## Beschreibung

Die Erfindung betrifft ein chirurgisches Instrument mit einer Push-Push-Verriegelungsmechanik mit den Merkmalen des Oberbegriffs des Anspruchs 1. Die Push-Push-Verriegelungsmechanik dient dazu, das chirurgische Instrument lösbar in einer betätigten Stellung zu halten. Das chirurgische Instrument ist zu einer Verwendung in der minimal-invasiven Chirurgie vorgesehen. Entriegelt wird die Push-Push-Verriegelungsmechanik durch eine kurze Bewegung in derselben Richtung, die zum Verriegeln der Push-Push-Verriegelungsmechanik geführt hat. Diese Richtung kann auch als Verriegelungsrichtung bezeichnet werden. Push-Push-Verriegelungsmechaniken sind beispielsweise von Kugelschreibern bekannt und werden aufgrund einer Form eines Schließstücks teilweise auch als Herzkurvenmechaniken bezeichnet. Push-Push-Verriegelungsmechaniken können sehr unterschiedliche Konstruktionen und Geometrien aufweisen, die nicht unbedingt wie eine Herzkurve aussehen.

Ein derartiges chirurgisches Instrument mit einer Push-Push-Verriegelungsmechanik offenbart die europäische Patentanmeldung EP 2 792 319 A1. Dieses chirurgische Instrument weist einen rohrförmigen Hohlschaft und eine in dem Hohlschaft verschiebliche Seele zu einer Betätigung des Instruments auf. "Betätigen" bedeutet ein Schwenken und insbesondere ein Schließen von beispielsweise Backen einer Zange oder Klingen einer Schere durch die Verschiebung der Seele im Hohlschaft. Die Aufzählung ist beispielhaft und nicht abschließend. Die Backen, Klingen oder dergleichen sind an einem Griffen des chirurgischen Instruments fernen Ende des Hohlschafts angeordnet. Zur Betätigung weist das bekannte chirurgische Instrument zwei Griffe auf, die symmetrisch zum Hohlschaft V-förmig in einem spitzen Winkel zueinander angeordnet sind und die zur Betätigung des chirurgischen Instruments durch Schwenken um einen Scheitelpunkt des V einander näherbar sind.

Die beiden Griffe des bekannten chirurgischen Instruments sind als Halbzylinderschalen ausgebildet und weisen an ihren einander zugewandten Hohl- und Innenseiten ein Wippenpaar mit einer Rastwippe und einer Sperrwippe auf, die schwenkbar an den Hohl- und Innenseiten der Griffe gelagert sind. Schwenkachsen der beiden Wippen verlaufen parallel zu einer Schwenkachse der Griffe und senkrecht zu einer von den beiden Griffen aufgespannten Ebene, die zugleich eine Bewegungsebene der Griffe ist, in der sich die Griffe bewegen. Wird das bekannte chirurgische Instrument betätigt indem seine beiden Griffe einander genähert werden, gelangt die Rastwippe in Eingriff mit der Sperrwippe und hält die Griffe in der einander genäherten Stellung und dadurch das chirurgische Instrument in einer betätigten Stellung. Zum Lösen werden die Griffe erneut einander ein kurzes Stück genähert, wodurch die Rastwippe außer Eingriff von der Sperrwippe gelangt. Die beiden Wippen, die durch Näherung in Eingriff miteinander gelangen und durch eine erneute, kurze Näherung wieder außer Eingriff voneinander gelangen, können auch als Push-Push-Verriegelungsmechanik aufgefasst werden.

Die Offenlegungsschrift DE 103 14 072 A1 offenbart ein chirurgisches Instrument mit zwei zangenartigen Griffen, die um eine Schwenkachse gegeneinander verschwenkbar sind. Zu einer Verriegelung in einer betätigten Stellung weist das chirurgische Instrument eine Push-Push-Verriegelungsmechanik mit einer Falle und einem Schließstück auf. Die Falle ist an einem Haltearm angeordnet, der um eine zu der Schwenkachse der beiden Griffe parallele Schwenkachse schwenkbar an einem der beiden Griffe angeordnet ist und der in Richtung eines anderen der beiden Griffe von dem einen Griff absteht. Das Schließstück ist fest in dem anderen Griff angeordnet. Eine vorgespannte Schraubendruckfeder beaufschlagt den Haltearm in eine Mittelstellung, aus der er beim Ver- und Entriegeln der Push-Push-Verriegelungsmechanik unter Erhöhung einer Federspannung der Schraubendruckfeder in beiden Richtungen auslenkbar ist.

Das Patent DE 198 41 249 C1 offenbart eine chirurgische Pinzette oder Zange mit einer Push-Push-Verriegelungsmechanik. Eine Falle ist als hakenförmige Umbiegung an einem Ende einer blattfederartigen Rückstellfeder ausgeführt, deren anderes Ende starr an einer Innenseite eines Griffs der Pinzette oder Zange befestigt ist. Ein Schließstück der Push-Push-Verriegelungsmechanik ist als kurzer Stift ausgeführt, der radial von einem Haltestift absteht, der von der Innenseite eines anderen Griffs der Pinzette oder Zange absteht und der zur Stabilisierung beim Zusammendrücken der beiden Griffe in eine Bohrung in dem anderen Griff taucht.

Aufgabe der Erfindung ist, ein chirurgisches Instrument mit einer alternativen Push-Push-Verriegelungsmechanik vorzuschlagen.

Gelöst wird diese Aufgabe durch ein chirurgisches Instrument mit den Merkmalen des Anspruchs 1. Das erfindungsgemäße chirurgische Instrument ist insbesondere zur Verwendung in der minimal-invasiven Chirurgie vorgesehen. Es weist einen Griff und einen Gegengriff auf, die zu einer Betätigung des chirurgischen Instruments in Bezug zueinander beweglich sind. Insbesondere sind der Griff und der Gegengriff einander näherbar und voneinander entfernbar. Andere Bewegungsmöglichkeiten des Griffs in Bezug zum Gegengriff schließt die Erfindung nicht aus. Der Griff und/oder der Gegengriff können schwenkbar und/oder verschiebbar sein. Zur Betätigung des chirurgischen Instruments wird der Griff in Bezug auf den Gegengriff bewegt, insbesondere wird zur Betätigung des chirurgischen Instruments der Griff dem Gegengriff genähert. Der Gegengriff kann ebenfalls ein Griff oder beispielsweise ein Gehäuse, Grundteil, Schaft oder ein sonstiges Teil des chirurgischen Instruments sein.

Beispielsweise weist das chirurgische Instrument eine Zange oder eine Schere auf, deren Backen oder Klingen bei der Betätigung einander genähert und vorzugsweise geschlossen werden. Die Bewegung des Griffs in Bezug auf den Gegengriff wird beispielsweise über eine Hebelmechanik oder ein Zahnstangengetriebe auf beispielsweise eine in einem Hohlschaft verschiebliche Seele oder auf zwei parallele, gegeneinander verschiebliche Schäfte übertragen, die die Backen der Zange oder Klingen der Schere verschwenken.

Außerdem weist das erfindungsgemäße chirurgische Instrument eine Push-Push-Verriegelungsmechanik zu einem lösbaren Halten des chirurgischen Instruments in einer betätigten Stellung auf. Die Push-Push-Verriegelungsmechanik weist eine Falle und ein Schließstück auf, das die Falle in einer verriegelten Stellung hintergreift. In der verriegelten Stellung der Push-Push-Verriegelungsmechanik ist das chirurgische Instrument betätigt und wird von der Push-Push-Verriegelungsmechanik in der betätigten Stellung gehalten. Zu einem Lösen bzw. Entriegeln der Push-Push-Verriegelungsmechanik wird der Griff erneut dem Gegengriff ein kurzes Stück genähert oder allgemein in der Verriegelungsrichtung bewegt, wodurch die Falle vom Schließstück frei kommt und die Push-Push-Verriegelungsmechanik entriegelt ist. Ist die Push-Push-Verriegelungsmechanik entriegelt, lässt sich das chirurgische Instrument wieder in eine nicht betätigte Ausgangsstellung zurück bewegen oder bewegt sich beispielsweise federbeaufschlagt von selbst zurück in die nicht betätigte Ausgangsstellung. Die Bezeichnung der Teile der Push-Push-Verriegelungsmechanik als Falle und Schließstück ist an ein Türschloss angelehnt.

Um in Eingriff mit und außer Eingriff von dem Schließstück treten zu können, ist die Falle der Push-Push-Verriegelungsmechanik des erfindungsgemäßen chirurgischen Instruments in zwei entgegengesetzten Richtungen auslenkbar. Ist die Falle ausgelenkt, kann sie zum Verriegeln und zum Entriegeln am Schließstück vorbei treten. Beim Verriegeln tritt die Falle mit dem Schließstück in Eingriff mit und beim Entriegeln tritt die Falle außer Eingriff von dem Schließstück beziehungsweise kommt von dem Schließstück frei. Eine Rückstellfeder wird beim Auslenken der Falle unabhängig von der Auslenkrichtung gespannt und beaufschlagt die ausgelenkte Falle entgegen ihrer Auslenkung zurück in eine nicht ausgelenkte Grundstellung. Beim Bewegen des Griffs in Bezug auf den Gegengriff zum Betätigen des chirurgischen Instruments wird die Falle ausgelenkt, tritt an dem Schließstück vorbei und wird von der Rückstellfeder, die beim Auslenken der Falle gespannt wird, entgegen der Auslenkung in eine Verriegelungsstellung bewegt, in der die Falle in Eingriff am Schließstück gelangt, was auch als Hintergreifen des Schließstücks von der Falle aufgefasst werden kann. Die Push-Push-Verriegelungsmechanik ist verriegelt und hält das chirurgische Instrument in der betätigten Stellung.

Eine Schwenkachse der Falle befindet sich in einer Bewegungsebene, in der sich der Griff bei seiner Bewegung in Bezug auf den Gegengriff bewegt, oder in einer zu der Bewegungsebene des Griffs parallelen Ebene. Und/oder die Schwenkachse der Falle verläuft tangential zu einem zur Schwenkachse des Griffs konzentrischen Kreis. Die Falle bewegt sich quer zu der Bewegungsebene des Griffs.

Zum Entriegeln der Push-Push-Verriegelungsmechanik wird der Griff erneut ein kurzes Stück in der Betätigungsrichtung bewegt, wobei die Falle wieder und vorzugsweise in der entgegengesetzten Richtung wie bei der Verriegelung ausgelenkt wird und von dem Schließstück frei kommt beziehungsweise außer Eingriff von dem Schließstück gelangt. Bei einer anschließenden Umkehr der Bewegungsrichtung des Griffs tritt die ausgelenkte Falle an dem Schließstück vorbei und das chirurgische Instrument gelangt zurück in eine nicht betätigte Grundstellung. Die Rückstellfeder bewegt die Falle zurück in die nicht ausgelenkte Grundstellung, so dass das chirurgische Instrument erneut betätigt werden kann, wobei die Push-Push-Verriegelungsmechanik das Instrument wieder selbsttätig in der betätigten Stellung verriegelt.

Die Falle und die Rückstellfeder sind verschiedene Teile. Beispielsweise ist die Falle einstückiger Bestandteil eines Hebels oder - insbesondere starr - an dem Hebel angeordnet, der schwenkbar an dem Griff, dem Gegengriff oder dem Fallenhalter gelagert ist. Die Rückstellfeder kann parallel zu dem Hebel, beispielsweise neben dem Hebel angeordnet sein.

Vorzugsweise sind die Falle an dem Griff und/oder das Schließstück an dem Gegengriff angeordnet oder umgekehrt, so dass sich die Falle und das Schließstück nähern wenn der Griff und der Gegengriff einander genähert werden, bis die Falle in Hintergriff am beziehungsweise in Eingriff mit dem Schließstück gelangt und verhindert, dass der Griff und der Gegengriff voneinander entfernt werden. Der Eingriff der Falle mit dem Schließstück bedeutet, dass die Push-Push-Verriegelungsmechanik verriegelt ist, wobei die verriegelte Push-Push-Verriegelungsmechanik den Griff und den Gegengriff bzw. das chirurgische Instrument in der betätigten Stellung hält. Zum Lösen bzw. Entriegeln der Push-Push-Verriegelungsmechanik müssen der Griff und der Gegengriff einander etwas weiter genährt werden, wodurch die Falle wieder außer Eingriff vom Schließstück gelangt, so dass der Griff und der Gegengriff anschließend auseinander bewegt werden können.

Zu einer platzsparenden Unterbringung sieht eine Ausgestaltung der Erfindung eine Anordnung der Falle und/oder des Schließstücks zwischen dem Griff und dem Gegengriff vor. Vorzugsweise stehen die Falle und/oder das Schließstück nicht seitlich über den Griff oder den Gegengriff über.

Ein bevorzugte Ausgestaltung der Erfindung sieht vor, dass die Falle schwenkbar an einem Fallenhalter angeordnet ist, an dem auch die Rückstellfeder angeordnet ist. Der Fallenhalter, die Falle und die Rückstellfeder bilden eine Baugruppe, die an dem Griff oder dem Gegengriff angeordnet ist. Diese Ausgestaltung der Erfindung ist auch möglich mit einer mit der Rückstellfeder einstückigen Falle.

Eine bevorzugte Ausgestaltung der Erfindung sieht vor, dass die Falle oder der Fallenhalter und das Schließstück fluchtende Befestigungslöcher zu ihrer Befestigung an dem Griff und an dem Gegengriff aufweisen. Das ist ein Vorteil, wenn der Gegengriff ein mit dem Griff baugleicher Griff ist, weil der Griff und der Gegengriff gleiche Befestigungslöcher für die Falle beziehungsweise den Fallenhalter und für das Schließstück aufweisen können und keine unterschiedlichen Befestigungen für die Falle beziehungsweise den Fallenhalter und das Schließstück brauchen. Mit "fluchtend" ist auch eine Anordnung der Befestigungslöcher in der Falle beziehungsweise dem Fallenhalter und in dem Schließstück auf einem Kreisbogen um eine Schwenkachse des Griffs und gegebenenfalls des Gegengriffs gemeint.

Eine Ausgestaltung der Erfindung sieht eine in ungespannter Stellung gerade Rückstellfeder vor. Beispielsweise ist die Rückstellfeder ein in ungespannter Stellung gerader Federdraht oder eine in ungespannter Stellung gerade Blattfeder.

Eine Ausgestaltung der Erfindung sieht ein Widerlager für die Falle an oder nahe an einer Eingriffsstelle mit dem Schließstück vor. "Nahe" bedeutet einen Abstand von vorzugsweise weniger als 1 mm und maximal wenigen Millimetern. Jedenfalls befindet sich das Widerlager näher an der Eingriffsstelle der Falle mit dem Schließstück als an einer Schwenkachse der Falle. Das Widerlager stützt die Falle in der Betätigungsrichtung des Griffs beziehungsweise in der Verriegelungsrichtung der Push-Push-Verriegelungsmechanik ab, also gegen ein Entfernen des Griffs vom Gegengriff. Bei verriegelter Push-Push-Verriegelungsmechanik stützt das Widerlager die Falle an oder nahe an der Eingriffsstelle mit dem Schließstück gegen ein Entfernen des Griffs vom Gegengriff ab, so dass das chirurgische Instrument stabil und mit niedriger Belastung der Falle in betätigter Stellung gehalten wird.

Eine Weiterbildung der Erfindung sieht eine Schiebeführung in einer Radialebene zur Schwenkachse der Falle als Widerlager vor. Die Schiebeführung kann beispielsweise ein Schlitz oder auch ein Widerlager auf einer Seite der Falle sein, der/das in einer Radialebene zur Schwenkachse der Falle verläuft.

Eine Weiterbildung der Erfindung sieht vor, dass die Push-Push-Verriegelungsmechanik mehrere Fallen und/oder Schließstücke aufweist, die in der Betätigungsrichtung des Griffs und/oder in der Verriegelungsrichtung der Push-Push-Verriegelungsmechanik versetzt zueinander angeordnet sind, so dass sich das chirurgische Instrument in verschieden weit betätigten Stellungen mit der Push-Push-Verriegelungsmechanik verriegeln lässt. Weist das chirurgische Instrument beispielsweise eine Zange auf, lässt sich dadurch eine Öffnungsweite von Backen der Zange und/oder eine Klemmkraft der Zange in Stufen einstellen.

Eine Ausgestaltung der Erfindung sieht ein hakenförmiges Schließstück und eine Schrägfläche auf einer Außenseite eines Hakens des Schließstücks vor, die eine zu der Bewegungsebene des Griffs in Bezug auf den Gegengriff parallele Ebene durch die Falle in der verriegelten Stellung in einem vorzugsweise spitzen Winkel schneidet. Der Haken kann als Schließstück im eigentlichen Sinn aufgefasst werden. Wird der Griff zum Entriegeln ein kurzes Stück weiter in der Betätigungsrichtung bewegt, kommt die Falle aus dem Haken des Schließstücks frei und gelangt, wenn anschließend der Griff entgegen der Betätigungsrichtung bewegt wird, auf die Schrägfläche auf der Außenseite des Hakens des Schließstücks. Bei einer weiteren Bewegung des Griffs entgegen der Betätigungsrichtung gleitet die Falle auf der Schrägfläche des Hakens des Schließstücks entlang und wird von der Schrägfläche so ausgelenkt, dass die Falle an dem Haken vorbei tritt und der Griff beziehungsweise das chirurgische Element in seinen nicht betätigte Ausgangsstellung zurück bewegt werden kann.

Eine Ausgestaltung der Erfindung sieht eine Entriegelungseinrichtung für die Push-Push-Verriegelungsmechanik vor, mit der die Push-Push-Verriegelungsmechanik insbesondere manuell entriegelt werden kann. Diese Ausgestaltung ist beispielsweise sinnvoll, wenn die Push-Push-Verriegelungsmechanik mehrere Fallen und/oder mehrere Schließstücke aufweist, durch die sie in verschiedenen betätigten Stellungen verriegelt werden kann. Die Push-Push-Verriegelungsmechanik kann durch die Entriegelungseinrichtung aus jeder Verriegelungsstellung entriegelt werden und muss nicht über eine letzte Verriegelungsstellung hinweg bewegt werden, um sie zu entriegeln. Die Entriegelungseinrichtung ist beispielsweise auch sinnvoll, wenn sie die Push-Push-Verriegelungsmechanik entriegelt hält, das heißt eine Verriegelung in der betätigten Stellung des chirurgischen Instruments verhindert. Das chirurgische Instrument kann dadurch benutzt werden ohne in der betätigten Stellung zu verriegeln, was seine Handhabung in manchen Fällen vereinfacht. In anderen Fällen wird die Entriegelungseinrichtung nicht bedient, das heißt die Push-Push-Verriegelungsmechanik bleibt wirksam und verriegelt das chirurgische Element selbsttätig in der betätigten Stellung.

Die Erfindung wird nachfolgend anhand eines in der Zeichnung dargestellten Ausführungsbeispiels näher erläutert. Es zeigen:
- Figur 1: eine perspektivische Darstellung eines chirurgischen Instruments gemäß der Erfindung;
- Figur 2: eine vergrößerte perspektivische Darstellung einer Betätigungseinheit des chirurgischen Instruments aus Figur 2;
- Figur 3: erfindungsgemäße Einzelteile einer Push-Push-Verriegelungsmechanik des chirurgischen Instruments in perspektivischer Darstellung;
- Figur 4: eine Darstellung eines Ver- und Entriegelungsvorgangs der Push-PushVerriegelungsmechanik des erfindungsgemäßen chirurgischen Instruments;
- Figur 5: die Einzelteile aus Figur 3 in betätigter beziehungsweise verriegelter Stellung;
- Figur 6: eine abgewandelte, erfindungsgemäße Ausführungsform einer Falle der Push-Push-Verriegelungsmechanik des chirurgischen Instruments; und
- Figur 7: eine abgewandelte Ausführungsform des chirurgischen Instruments aus Figur 1 in perspektivischer Darstellung.

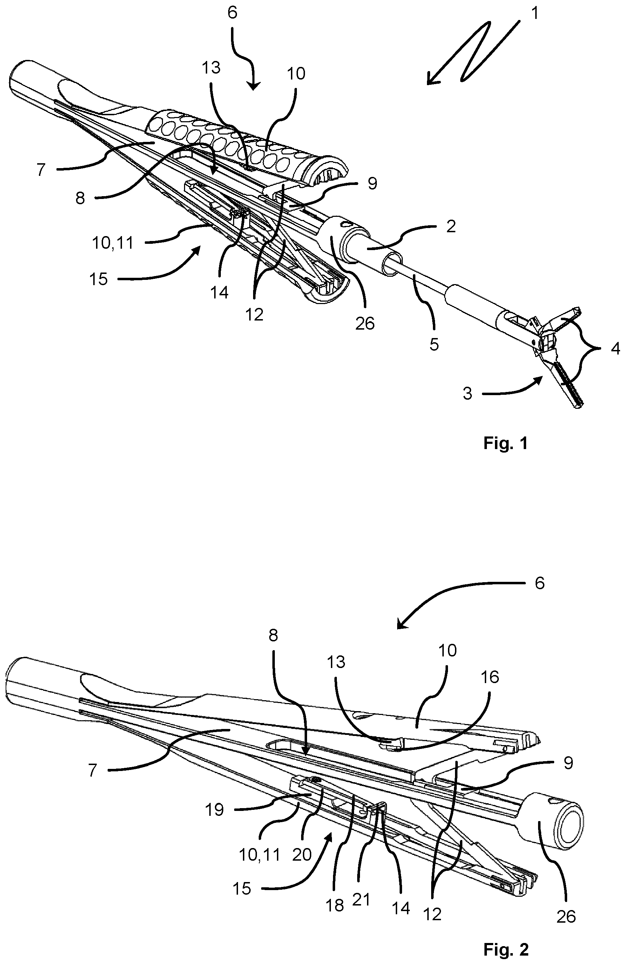

Das in Figur 1 dargestellte chirurgische Instrument 1 ist zu einer Verwendung in der minimal-invasiven Chirurgie vorgesehen. Es weist einen rohrförmigen Hohlschaft 2 mit einer Zange 3 an einem Ende auf. Die Zange 3 weist zwei schwenkbare Backen 4 auf, die mit einem Draht als Seele 5, die in dem Hohlschaft 2 verschiebbar ist, zusammen- und auseinander schwenkbar sind. In Figur 1 ist ein Abschnitt des Hohlschafts 2 heraus geschnitten damit die Seele 5 sichtbar ist. Tatsächlich geht der Hohlschaft 2 durch. Anstelle der Zange 3 kann das chirurgische Instrument 1 beispielsweise eine Schere mit zwei schwenkbaren Klingen aufweisen (nicht dargestellt). Die Erfindung ist nicht auf eine Zange 3 oder Schere beschränkt. Die Zange 3 oder Schere kann auch nur eine schwenkbare Backe 4 oder Klinge und eine feststehende Backe oder Klinge aufweisen. Anstelle des Hohlschafts 2 und der Seele 5 kann das chirurgische Instrument 1 beispielsweise auch zwei zueinander parallele, insbesondere aneinander anliegende und in ihrer Längsrichtung gegeneinander verschiebliche Schäfte aufweisen, die beide verschieblich oder von denen einer verschieblich und der andere feststehend ist (nicht dargestellt).

An einem der Zange 3 fernen Ende des Hohlschafts 2 weist das chirurgische Instrument 1 eine Betätigungseinheit 6 mit einem Grundhalter 7 auf, die in Figur 2 als Einzelteil dargestellt ist. Der Grundhalter 7 ist im Ausführungsbeispiel streifenförmig mit einem Langloch 8 in einem Mittelbereich. An einem Ende weist der Grundhalter 7 ein Rohrstück 26 auf, in dem das Ende des Hohlschafts 2 lösbar und wechselbar befestigt ist.

In dem Langloch 8 des Grundhalters 7 ist ein hier als Gleitstein 9 bezeichnetes bezeichnetes Teil koaxial zum Hohlschaft 2 verschiebbar geführt, an beziehungsweise in dem die Seele 5 lösbar und wechselbar befestigt ist.

Die Betätigungseinheit 6 weist zwei Griffe 10 auf, die in der dargestellten, nicht betätigten Stellung des chirurgischen Instruments 1 V-förmig schräg auseinander stehen und die zur Betätigung des chirurgischen Instruments 1 zusammen gedrückt werden können, bis sie parallel zueinander verlaufend an dem streifenförmigen Grundhalter 7 anliegen. Es ist nicht notwendig, dass die beiden Griffe 10 bis zur Anlage an dem Grundhalter 7 zusammendrückbar sind, sondern es genügt, dass sich ein Winkel des V, in dem die beiden Griffe 10 zueinander stehen, verkleinern oder jedenfalls verändern lässt. Möglich sind auch Griffe 10, die anders als durch Schwenken beweglich sind. Im Ausführungsbeispiel sind die beiden Griffe 10 halbzylinder- beziehungsweise teilzylinderschalenförmig.

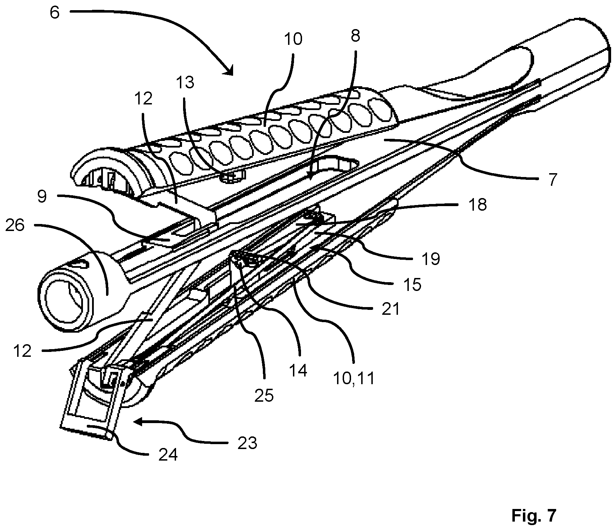

Die Griffe 10 sind in Figur 1 mit Griffschalen mit einem Lochmuster und in Figuren 2 und 7 ohne die Griffschalen gezeichnet.

Die beiden Griffe 10 sind an einem Ende nach Art von Blattfedern ausgebildet und an dem Grundhalter 7 der Betätigungseinheit 6 festgelegt, so dass sie schwenkbar sind und in die dargestellte V-Stellung schwenken, wenn sie nicht mit einer Kraft beaufschlagt werden. Im Ausführungsbeispiel sind die beiden Griffe 10 an einem dem Hohlschaft 2 fernen Ende des Grundhalters 7 der Betätigungseinheit 6 befestigt, sie können allerdings auch an dem Ende des Grundhalters 7 befestigt sein, an dem der Hohlschaft 2 befestigt ist (nicht dargestellt), so dass sich ein Scheitel des V, das die beiden Griffe 10 bilden, an dem Ende des Grundhalters 7 befindet, an dem der Hohlschaft 2 befestigt ist, anstatt an dem dem Hohlschaft 2 fernen Ende, wie dargestellt.

Eine weitere Möglichkeit ist eine Betätigungseinheit mit nur einem beweglichen Griff 10, wobei dann der Grundhalter 7 der Betätigungseinheit 6 oder ein feststehender Griff einen Gegengriff 11 bildet (nicht dargestellt). Zur Unterscheidung kann einer der beiden Griffe 10 als Gegengriff 11 bezeichnet werden, wobei vorzugsweise der schwenkbare oder allgemein der bewegliche Griff 10 als Griff 10 und der andere Griff 10 als Gegengriff 11 bezeichnet wird, unabhängig davon, ob er beweglich oder feststehend ist.

Eine weitere, nicht dargestellte Möglichkeit ist eine nach Art eines Pistolengriffs abgewinkelte Anordnung der Betätigungseinheit 6 am Hohlschaft 2 (nicht dargestellt), die allerdings eine Winkelumlenkung zum Verschieben der Seele 5 im Hohlschaft 2 zur Betätigung des chirurgischen Instruments 1 benötigt.

Zum Verschieben der Seele 5 im Hohlschaft 2 weist die Betätigungseinheit 6 zwei Schwenkhebel 12 auf, deren einen Enden schwenkbar an den Griffen 10 und deren andere Enden schwenkbar an dem Gleitstein 9 angeordnet sind. Die beiden Schwenkhebel 12 stehen von dem Grundhalter 7 V-förmig in einem stumpferen Winkel als die Griffe 10 auseinander. Durch Zusammendrücken der Griffe 10 werden die Schwenkhebel 12 zusammengedrückt und verschieben dabei den Gleitstein 9 und die Seele 5, die lösbar an dem Gleitstein 9 befestigt ist. Die Verschieberichtung ist so gewählt, dass beim Zusammendrücken der Griffe 10 die Backen 4 der Zange 3 zusammen schwenken. Diese Bewegungsrichtung wird hier als Betätigungsrichtung beziehungsweise als Betätigung des chirurgischen Instruments 1 bezeichnet. Indem die beiden Schwenkhebel 12 umgekehrt V-förmig angeordnet werden als dargestellt, das heißt ein Scheitel des V's der Schwenkhebel 12 ist dem Hohlschaft 2 zu- anstatt abgewandt, lässt sich die Verschieberichtung des Gleitsteins 9 beim Zusammendrücken der Griffe 10 umkehren (nicht dargestellt). Im Ausführungsbeispiel sind die Schwenkhebel 12 nahe an freien, voneinander entfernten Enden der Griffe 10 angeordnet, was allerdings nicht zwingend für die Erfindung ist. Durch Versetzen der Schwenkhebel 12 in Längsrichtung der Griffe 10 lassen sich Hebelverhältnisse der Betätigungseinheit 6 des erfindungsgemäßen chirurgischen Instruments 1 ändern.

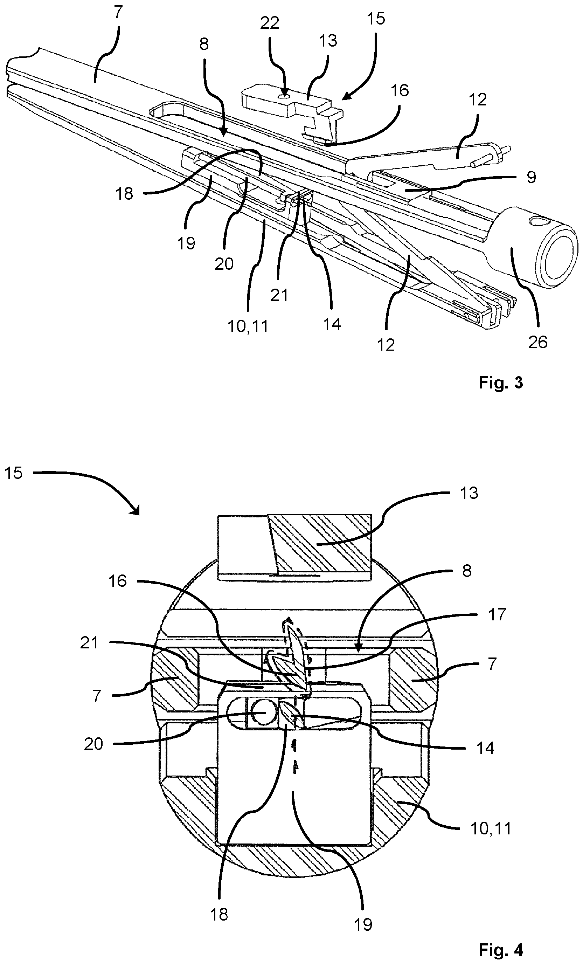

An einander und dem Grundhalter 7 der Betätigungseinheit 6 zugewandten Innenseiten der Griffe 10 sind an einem der beiden Griffe 10 ein Schließstück 13 und dem Schließstück 13 gegenüber am anderen Griff 10, der hier auch als Gegengriff 11 bezeichnet wird, eine Falle 14 angeordnet. Figur 3 zeigt das Schließstück 13 und die Falle 14 als Einzelteile in der Stellung zueinander, die sie bei nicht betätigtem chirurgischen Instrument 1 aufweisen, die die Figuren 1 und 2 zeigen. In dieser Stellung ist die Push-Push-Verriegelungsmechanik 15 entriegelt. Die Bezeichnung als "Schließstück" und als "Falle" sind einem Türschloss entlehnt. Das Schließstück 13 und die Falle 14 bilden eine Push-Push-Verriegelungsmechanik 15 oder Teile der Push-Push-Verriegelungsmechanik 15 des chirurgischen Instruments 1 gemäß der Erfindung.

Die Falle 14 weist einen linsenförmigen, schräg zu einer Bewegungsebene der Griffe 10 und schräg zu einer Auslenkungsrichtung der Falle 10 stehenden Querschnitt auf und ist einstückig an einem Ende eines Hebels 18 angeordnet, der schwenkbar an einem Fallenhalter 19 gelagert ist. Eine Schwenkachse des Hebels 18 verläuft in oder parallel zu der Bewegungsebene der Griffe 10 beziehungsweise tangential zu einem zu einer Schwenkachse der Griffe 10 konzentrischen Kreis. Der Hebel 18 ist dadurch quer zur Bewegungsebene der Griffe 10 in beiden Richtungen schwenkbar und die Falle 14 quer zur Bewegungsebene der Griffe 10 in beiden Richtungen auslenkbar. Denkbar sind auch Ausführungen, bei denen das Schließstück 13 zusätzlich zur Falle 14 oder auch ausschließlich in beiden Richtungen schwenkbar ist.

Parallel neben dem Hebel 18 ist ein Federdraht als Rückstellfeder 20 der Falle 14 angeordnet, dessen der Falle 14 fernes Ende am Fallenhalter 19 und dessen der Falle 14 nahes Ende an dem Hebel 18 festgelegt ist. In entspannter Stellung ist der die Rückstellfeder 20 bildende Federdraht gerade, was allerdings nicht zwingend für die Erfindung ist. Wird die Falle 14 ausgelenkt, egal in welcher Richtung, spannt sie die Rückstellfeder 20, die die Falle 14 in eine nicht aus gelenkte Grundstellung zurück bewegt, wenn sie frei beweglich ist.

Das die Falle 14 aufweisende Ende des Hebels 18 durchgreift einen in der Schwenkrichtung des Hebels 18 und in der Auslenkrichtung der Falle 14 verlaufenden Schlitz im Fallenhalter 19, der eine Schiebeführung für die Falle 14 in deren Auslenkrichtung und ein Widerlager 21 für die Falle 14 an oder nahe einer Eingriffsstelle mit dem Schließstück 13 bildet.

Das Schließstück 13 und der Fallenhalter 19 weisen miteinander fluchtende Befestigungslöcher 22 auf, an denen sie mit den Griffen 10 verschraubt, das heißt an den Griffen 10 befestigt sind. Genau genommen befinden sich die Befestigungslöcher 22 des Schließstücks 13 und des Fallenhalters 19 auf einem Kreisbogen um die Schwenkachse der Griffe 10. Die beiden Griffe 10 können dadurch gleich ausgebildet sein.

Der Fallenhalter 19 mit dem die Falle 14 aufweisenden, schwenkbaren Hebel 18 und die Rückstellfeder 20 bildet eine vormontierbare Baugruppe, die als Ganzes an der Innenseite eines der beiden Griffe 10 befestigt werden kann.

Das Schließstück 13 weist einen in Richtung der Falle 14 abstehenden Haken 16 mit einer Schrägfläche 17 an einer Außenseite des Hakens 16 auf, die in Figur 4 zu sehen ist, die einen Querschnitt durch den Haken 16 des Schließstücks 13 und eine Stirnansicht der linsenförmigen, schräg stehenden Falle 14 zeigt. Die Schrägfläche 17 kreuzt die Bewegungsebene der Griffe 10 oder eine zu der Bewegungsebene der Griffe 10 parallele Ebene, in der sich die Falle 14 befindet, wenn sie nicht ausgelenkt ist, in einem spitzen Winkel. Der Haken 16 kann auch als das Schließstück 13 im eigentlichen Sinn aufgefasst werden.

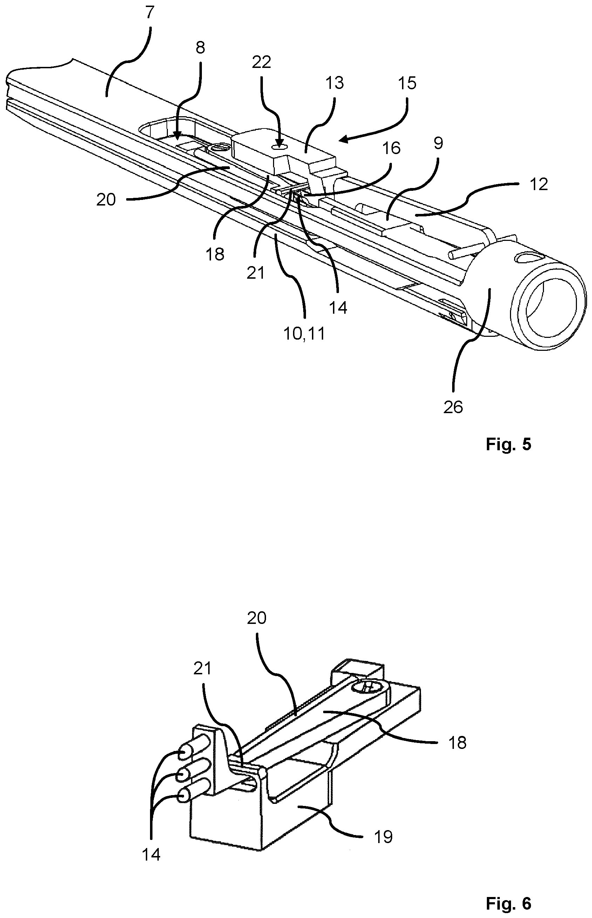

Werden die beiden Griffe 10 zu einer Betätigung des chirurgischen Instruments 1 zusammengedrückt, nähert sich die Falle 14 wie in Figur 4 dargestellt dem Haken 16 des Schließstücks 13 in der Bewegungsebene der Griffe 10 oder der zu der Bewegungsebene der Griffe 10 parallelen Ebene, bis die Falle 14 von außen gegen den Haken 16 stößt. Werden die Griffe 10 weiter zusammengedrückt, lenkt der Haken 16 die Falle 14 aus, so dass die Falle 14 am Haken 16 vorbei treten kann, und spannt dabei die Rückstellfeder 20. Das Vorbeitreten der Falle 14 am Haken 16 des Schließstücks 13 kann auch als Vorbeitreten der Falle 14 am Schließstück 13 aufgefasst werden. Wenn die Falle 14 am Schließstück 13 beziehungsweise an dessen Haken 16 vorbei getreten ist, bewegt die gespannte Rückstellfeder 20 die Falle 14 entgegen ihrer Auslenkung zurück in Richtung ihrer nicht ausgelegten Grundstellung, so dass die Falle 14 in den Haken 16 gelangt, was als Eingriff der Falle 14 mit dem Schließstück 13 aufgefasst werden kann. Diese Stellung, die hier als verriegelte Stellung der Falle 14 beziehungsweise als verriegelte Stellung der Push-Push-Verriegelungsmechanik 15 bezeichnet wird, ist in Figur 4 mit Strichlinien und in Figur 5 gezeichnet. In der verriegelten Stellung hält die Push-Push-Verriegelungsmechanik 15 die beiden Griffe 10 in der zusammengerückten, das heißt zum Grundhalter 7 geschwenkten Stellung und hält damit das chirurgische Instrument 1 in der betätigten Stellung. Die Backen 4 der Zange 3 sind zusammen gedrückt.

Der Schlitz im Fallenhalter 19, der das Widerlager 21 für die Falle 14 bildet, stützt die bei verriegelter Push-Push-Verriegelungsmechanik 15 mit dem Haken 16 des Schließstück 13 in Eingriff stehende Falle 14 in einer Schließrichtung, das heißt gegen ein Auseinanderbewegen der Griffe 10 ab. Das Widerlager 21 stützt die Falle 14 nahe ihrer Eingriffstelle mit dem Haken 16 des Schließstücks 13 und damit nahe ihrer Eingriffsstelle am Schließstück 13 ab, wodurch die verriegelte Falle 14 gut abgestützt und der Hebel 18 entlastet ist.

Zum Entriegeln der Push-Push-Verriegelungsmechanik 15 und Lösen des chirurgischen Instruments 1 aus der betätigten Stellung werden die beiden Griffe 10 noch einmal ein kurzes Stück zusammengedrückt, so dass die Falle 14, wie in Figur 4 zu sehen, aus dem Haken 16 des Schließstücks 13 gehoben wird. Sobald sich die Falle 14 über dem Haken 16 befindet, schwenkt die immer noch oder wieder gespannte Rückstellfeder 20 die Falle 14 in ihre nicht ausgelenkte Grundstellung. Werden die Griffe 10 jetzt losgelassen, federn sie aufgrund ihrer Ausbildung als Blattfedern auseinander. Dabei gelangt die Falle 14 auf die Schrägfläche 17 auf der Außenseite des Hakens 16, die die Falle 14 in der entgegengesetzten Richtung wie beim Schließen und Verriegeln der Push-Push-Verriegelungsmechanik 15 auslenkt und dabei die Rückstellfeder 20 spannt. Die Falle 14 tritt wieder, beim Lösen in entgegengesetzter Richtung wie beim Schließen beziehungsweise Verriegeln am Haken 16 des Schließstücks 13 und damit am Schließstück 13 vorbei, die Griffe 10 schwenken auseinander in ihre ursprüngliche Grundstellung, verschieben über die Schwenkhebel 12 und den Gleitstein 9 die Seele 5 im Hohlschaft 2 und öffnen dabei die Backen 4 der Zange 3. Wenn die Falle 14 am Haken 16 des Schließstück 13 vorbei getreten ist, schwenkt die gespannte Rückstellfeder 20 die Falle 14 wieder zurück in ihre nicht ausgelenkte Grundstellung, so dass bei einem erneuten Zusammendrücken der Griffe 10 die Push-Push-Verriegelungsmechanik 15 das chirurgische Instrument 1 wie vorstehend beschrieben wieder in der betätigten Stellung verriegelt.

Figur 6 zeigt eine abgewandelte Baugruppe mit der Falle 14 schräg auf eine gegenüberliegende Seite wie in Figuren 1 bis 5: hier sind an dem Ende des Hebels 18 drei Fallen 14 in der Bewegungsrichtung der Griffe 10 versetzt zueinander angeordnet, wodurch die Push-Push-Verriegelungsmechanik 15 in drei Stellungen verriegelt werden kann. Es können dadurch die Backen 4 der Zange 3 unterschiedlich eng zusammen geschwenkt und/oder mit unterschiedlicher Klemmkraft gegeneinander gespannt gehalten werden. Möglich sind auch Ausführungen mit zwei oder mit mehr als drei Fallen 14 (nicht dargestellt). Zusätzlich oder anstelle mehrerer Fallen 14 kann das chirurgische Instrument 1 ein Schließstück mit mehreren in der Bewegungsrichtung der Griffe 10 versetzt zueinander angeordneten Haken 16 beziehungsweise mehreren in der Bewegungsrichtung der Griffe 10 zueinander versetzten Schließstücken 13 aufweisen (nicht dargestellt).

Eine Abwandlung des chirurgischen Instruments 1 zeigt Figur 7: dort weist das erfindungsgemäßen chirurgische Instrument 1 zusätzlich eine Entriegelungseinrichtung 23 auf, die insbesondere für die in Figur 6 gezeigte Ausführung mit mehreren Fallen 14 sinnvoll ist, weil die Push-Push-Verriegelungsmechanik 15 in jeder Verriegelungsstellung entriegelt werden kann und nicht bis über eine letzte Verriegelungsstellung hinweg betätigt werden muss, bevor sich die Push-Push-Verriegelungsmechanik 15 wie weiter oben beschrieben selbsttätig entriegelt. Sinnvoll ist die Entriegelungseinrichtung 23 auch, wenn das chirurgische Instrument 1 selbsttätig verriegelnd und nicht verriegelnd verwendbar sein soll.

Die Entriegelungseinrichtung 23 weist einen Entriegelungshebel 24 auf, der schwenkbar am freien Ende eines der beiden Griffe 10 angeordnet ist. Eine Anordnung des Entriegelungshebels 24 an anderer Stelle ist möglich (nicht dargestellt). Der Entriegelungshebel 24 ist - im Ausführungsbeispiel - mittels eines Blechstreifens 25 gelenkig mit dem Hebel 18 der Falle 14 verbunden. Der Blechstreifen 25 ist mit Abstand von der Schwenkachse gelenkig am Entriegelungshebel 24 und exzentrisch und gelenkig an einer Seite des Hebels 18 angeordnet, so dass durch Zug am Blechstreifen 25 der Hebel 18 zu einer Seite und dadurch die Falle 14 in einer Richtung ausgelenkt wird. Die Auslenkrichtung ist wie beim Verriegeln, so dass die Falle 14, wenn sie in Eingriff mit dem Haken 16 des Schließstücks 13 und die Push-Push-Verriegelungsmechanik 15 verriegelt ist, die Falle 14 zur Seite aus dem Haken 16 frei kommt.

Der Zug am Blechstreifen 25 zum Entriegeln der Push-Push-Verriegelungsmechanik 15 wird durch Schwenken des Entriegelungshebels 24 erzeugt. Im Ausführungsbeispiel ist der Blechstreifen 25 so am Entriegelungshebel 24 angelenkt, dass beim Entriegeln die Anlenkstelle des Blechstreifens 25 am Entriegelungshebel 24 über eine gedachte Gerade durch die Anlenkstelle des Blechstreifens 25 am Hebel 18 der Falle 14 und durch die Schwenkachse des Entriegelungshebels 24 hinweg tritt. Das ermöglicht eine eigenstabile Entriegelungsstellung der Entriegelungseinrichtung, die die Push-Push-Verriegelungsmechanik 15 entriegelt hält, ohne dass der Entriegelungshebel 24 gehalten werden muss. Wird der Entriegelungshebel 24 weniger weit geschwenkt, ist die Entriegelung nicht stabil, sondern die Push-Push-Verriegelungsmechanik 15 verriegelt selbsttätig, wenn der Entriegelungshebel 24 losgelassen wird. Die Entriegelungseinrichtung kann auch ohne stabile Entriegelungsstellung ausgeführt werden.

Figur 7 zeigt das chirurgische Instrument 1 in einer perspektivischen Darstellung mit Blick wie in Figur 6 auf eine im Vergleich mit den Figuren 1 bis 5 gegenüberliegende Seite des Instruments 1, damit der dort befindliche Blechstreifen 25 der Entriegelungseinrichtung 23 sichtbar ist.

## Patentansprüche

1. Chirurgisches Instrument (1) zur Verwendung in der minimal-invasiven Chirurgie, mit einem Griff (10), der zu einer Betätigung des chirurgischen Instruments (1) in Bezug auf einen Gegengriff (11) beweglich ist, mit einer Push-Push-Verriegelungsmechanik (15) zu einem lösbaren Halten des chirurgisches Instruments (1) in einer betätigten Stellung, die eine schwenkbare Falle (14) und ein Schließstück (13) aufweist, das die Falle (14) in einer verriegelten Stellung hintergreift, so dass sie das chirurgische Instrument (1) lösbar in der betätigten Stellung hält, wobei die Falle (14) aus der verriegelten Stellung in zwei entgegengesetzten Richtungen auslenkbar ist, so dass die Falle (14) bei der Bewegung des Griffs (10) in Bezug auf den Gegengriff (11) an dem Schließstück (13) vorbeitreten und dadurch in den Eingriff mit und außer Eingriff von dem Schließstück (13) treten kann, wobei die Push-Push-Verriegelungsmechanik (15) eine Rückstellfeder (20) aufweist, die durch eine Auslenkung der Falle (14) gespannt wird und die die ausgelenkte Falle (14) unabhängig von ihrer Auslenkrichtung zurück in eine nicht ausgelenkte Stellung beaufschlagt, und wobei die Falle (14) und die Rückstellfeder (20) verschiedene Teile sind, **dadurch gekennzeichnet, dass** eine Schwenkachse der Falle (14) in einer Bewegungsebene des Griffs (10) in Bezug zu dem Gegengriff (11) verläuft und/oder tangential zu einem zu einer Schwenkachse des Griffs (10) konzentrischen Kreis verläuft.

2. Chirurgisches Instrument nach Anspruch 1, **dadurch gekennzeichnet, dass** die Falle (14) an dem Griff (10) oder dem Gegengriff (11) angeordnet ist und/oder dass das Schließstück (13) an dem Gegengriff (11) oder dem Griff (10) angeordnet ist.

3. Chirurgisches Instrument nach Anspruch 1 oder 2, **dadurch gekennzeichnet, dass** die Falle (14) und/oder das Schließstück (13) zwischen dem Griff (10) und dem Gegengriff (11) angeordnet sind.

4. Chirurgisches Instrument nach einem oder mehreren der Ansprüche 1 bis 3, **dadurch gekennzeichnet, dass** die Falle (14) und/oder eine die Falle (14) aufweisende Baugruppe und das Schließstück (13) fluchtende Befestigungslöcher (22) aufweisen.

5. Chirurgisches Instrument nach einem oder mehreren der vorhergehenden Ansprüche, **dadurch gekennzeichnet, dass** die Rückstellfeder (20) in ungespannter Stellung gerade ist.

6. Chirurgisches Instrument nach einem oder mehreren der vorhergehenden Ansprüche, **dadurch gekennzeichnet, dass** die Falle (14) ein Widerlager (21) an oder nahe an einer Eingriffstelle mit dem Schließstück (13) aufweist, das die Falle (14) in einer Schließrichtung abstützt.

7. Chirurgisches Instrument nach Anspruch 6, **dadurch gekennzeichnet, dass** die Falle (14) eine Schiebeführung in einer Radialebene zu ihrer Schwenkachse als Widerlager (21) aufweist.

8. Chirurgisches Instrument nach einem oder mehreren der vorhergehenden Ansprüche, **dadurch gekennzeichnet, dass** die Push-Push-Verriegelungsmechanik (15) mehrerer Fallen (14) und/oder Schließstücke (13) aufweist, die das chirurgische Instrument (1) in verschiedenen verriegelten Stellungen halten.

9. Chirurgisches Instrument nach einem oder mehreren der vorhergehenden Ansprüche, **dadurch gekennzeichnet, dass** das Schließstück (13) hakenförmig ist und eine Schrägfläche (17) auf einer Außenseite eines Hakens (16) aufweist, die eine zu der Bewegungsebene des Griffs (10) in Bezug auf den Gegengriff (11) parallele Ebene durch die Falle in der verriegelten Stellung in einem spitzen Winkel schneidet, so dass die Falle (14) nach einem Lösen der Push-Push-Verriegelungsmechanik (15) an der Schrägfläche (17) entlang gleitet und dabei seitlich am Haken (16) des Schließstücks (13) vorbei tritt.

10. Chirurgisches Instrument nach einem oder mehreren der vorhergehenden Ansprüche, **dadurch gekennzeichnet, dass** das chirurgische Instrument (1) eine Entriegelungseinrichtung (23) für die Push-Push-Verriegelungsmechanik (15) aufweist, mit der die Push-Push-Verriegelungsmechanik (15) entriegelbar ist.

11. Chirurgisches Instrument nach Anspruch 10, **dadurch gekennzeichnet, dass** die Entriegelungseinrichtung (23) eine stabile Entriegelungsstellung aufweist, in der sie die Push-Push-Verriegelungsmechanik (15) entriegelt hält.

## Claims

1. Surgical instrument (1) for use in minimally invasive surgery, having a handgrip (10) which is movable relative to a counter-grip (11) to actuate the surgical instrument (1), having a push-push locking mechanism (15) for releasably holding the surgical instrument (1) in an actuated position, which locking mechanism has a pivotable latch (14) and a closure piece (13) behind which the latch (14) engages in a locked position so that it holds the surgical instrument (1) releasably in the actuated position, the latch (14) being deflectable out of the locked position in two opposite directions, so that when the handgrip (10) moves relative to the counter-grip (11) the latch (14) is able to move past the closure piece (13) and thereby move into and out of engagement with the closure piece (13), wherein the push-push locking mechanism (15) has a restoring spring (20) which is tensioned by deflection of the latch (14) and which urges the deflected latch (14), irrespective of its direction of deflection, back into a non-deflected position, and wherein the latch (14) and the restoring spring (20) are different parts, **characterised in that** a pivot axis of the latch (14) runs in a plane of movement of the handgrip (10) relative to the counter-grip (11) and/or runs tangentially with respect to a circle concentric with a pivot axis of the handgrip (10).

2. Surgical instrument according to claim 1, **characterised in that** the latch (14) is arranged on the handgrip (10) or on the counter-grip (11) and/or the closure piece (13) is arranged on the counter-grip (11) or on the handgrip (10).

3. Surgical instrument according to claim 1 or 2, **characterised in that** the latch (14) and/or the closure piece (13) is/are arranged between the handgrip (10) and the counter-grip (11).

4. Surgical instrument according to one or more of claims 1 to 3, **characterised in that** the latch (14) and/or an assembly having the latch (14) and the closure piece (13) have aligned fixing holes (22).

5. Surgical instrument according to one or more of the preceding claims, **characterised in that** the restoring spring (20) is straight in the untensioned position.

6. Surgical instrument according to one or more of the preceding claims, **characterised in that** the latch (14) has a counter-bearing (21) at or close to a point of engagement with the closure piece (13), which counter-bearing supports the latch (14) in a closure direction.

7. Surgical instrument according to claim 6, **characterised in that** the latch (14) has a sliding guide in a radial plane with respect to its pivot axis as counter-bearing (21).

8. Surgical instrument according to one or more of the preceding claims, **characterised in that** the push-push locking mechanism (15) has a plurality of latches (14) and/or closure pieces (13) which hold the surgical instrument (1) in various locked positions.

9. Surgical instrument according to one or more of the preceding claims, **characterised in that** the closure piece (13) is hook-shaped and has a sloping surface (17) on an outer side of a hook (16), which surface intersects a plane through the latch in the locked position at an acute angle, that plane being parallel to the plane of movement of the handgrip (10) relative to the counter-grip (11), so that the latch (14), after release of the push-push locking mechanism (15), slides along the sloping surface (17) and in so doing moves laterally past the hook (16) of the closure piece (13).

10. Surgical instrument according to one or more of the preceding claims, **characterised in that** the surgical instrument (1) has an unlocking device (23) for the push-push locking mechanism (15), with which the push-push locking mechanism (15) is unlockable.

11. Surgical instrument according to claim 10, **characterised in that** the unlocking device (23) has a stable unlocking position in which it keeps the push-push locking mechanism (15) unlocked.

## Revendications

1. Instrument chirurgical (1) à usage dans la chirurgie mini-invasive, comprenant une poignée (10) qui est mobile par rapport à une poignée antagoniste (11) pour un actionnement de l'instrument chirurgical (1), comprenant un mécanisme de verrouillage de type push-push (15) pour maintenir de manière amovible l'instrument chirurgical (1) dans une position actionnée, qui présente un pêne (14) pouvant pivoter et une pièce de fermeture (13) qui vient en prise par l'arrière avec le pêne (14) dans une position verrouillée de sorte qu'il maintienne de manière amovible dans la position actionnée l'instrument chirurgical (1), dans lequel le pêne (14) peut être dévié hors de la position verrouillée dans deux directions opposées de telle sorte que le pêne (14) puisse passer le long de la pièce de fermeture (13) lors du déplacement de la poignée (10) par rapport à la poignée antagoniste (11) et peut ainsi venir en prise avec la pièce de fermeture (13) et hors prise d'avec celle-ci, dans lequel le mécanisme de verrouillage de type push-push (15) présente un ressort de rappel (20) qui est tendu par une déviation du pêne (14) et qui soumet à une action le pêne (14) dévié indépendamment de sa direction de déviation pour revenir dans une position non déviée, et dans lequel le pêne (14) et le ressort de rappel (20) sont des parties différentes, **caractérisé en ce qu'**un axe de pivotement du pêne (14) s'étend dans un plan de déplacement de la poignée (10) par rapport à la poignée antagoniste (11) et/ou s'étend de manière tangentielle par rapport à un cercle concentrique par rapport à un axe de pivotement de la poignée (10).

2. Instrument chirurgical selon la revendication 1, **caractérisé en ce que** le pêne (14) est disposé sur la poignée (10) ou la poignée antagoniste (11), et/ou que la pièce de fermeture (13) est disposée sur la poignée antagoniste (11) ou la poignée (10).

3. Instrument chirurgical selon la revendication 1 ou 2, **caractérisé en ce que** le pêne (14) et/ou la pièce de fermeture (13) sont disposés entre la poignée (10) et la poignée antagoniste (11).

4. Instrument chirurgical selon l'une ou plusieurs des revendications 1 à 3, **caractérisé en ce que** le pêne (14) et/ou un module présentant le pêne (14) et la pièce de fermeture (13) présentent des trous de fixation (22) alignés.

5. Instrument chirurgical selon l'une ou plusieurs des revendications précédentes, **caractérisé en ce que** le ressort de rappel (20) est droit dans une position non tendue.

6. Instrument chirurgical selon l'une ou plusieurs des revendications précédentes, **caractérisé en ce que** le pêne (14) présente une butée (21) sur un emplacement de prise ou à proximité de celui-ci avec la pièce de fermeture (13), qui soutient dans une direction de fermeture le pêne (14).

7. Instrument chirurgical selon la revendication 6, **caractérisé en ce que** le pêne (14) présente en tant que butée (21) un guide à glissière dans un plan radial par rapport à son axe de pivotement.

8. Instrument chirurgical selon l'une ou plusieurs des revendications précédentes, **caractérisé en ce que** le mécanisme de verrouillage de type push-push (15) présente plusieurs pênes (14) et/ou pièces de fermeture (13) qui maintiennent l'instrument chirurgical (1) dans différentes positions verrouillées.

9. Instrument chirurgical selon l'une ou plusieurs des revendications précédentes, **caractérisé en ce que** la pièce de fermeture (13) est en forme de crochet et présente une surface inclinée (17) sur un côté extérieur d'un crochet (16) qui coupe selon un angle aigu un plan parallèle au plan de déplacement de la poignée (10) par rapport à la poignée antagoniste (11) à travers le pêne dans la position verrouillée si bien que le pêne (14) glisse le long de la surface inclinée (17) après un desserrement du mécanisme de verrouillage de type push-push (15) et passe ce faisant latéralement le long du crochet (16) de la pièce de fermeture (13).

10. Instrument chirurgical selon l'une ou plusieurs des revendications précédentes, **caractérisé en ce que** l'instrument chirurgical (1) présente un dispositif de déverrouillage (23) pour le mécanisme de verrouillage de type push-push (15) avec lequel le mécanisme de verrouillage de type push-push (15) peut être déverrouillé.

11. Instrument chirurgical selon la revendication 10, **caractérisé en ce que** le dispositif de déverrouillage (23) présente une position de déverrouillage stable, dans laquelle il maintient déverrouillé le mécanisme de verrouillage de type push-push (15).
